# EUROPEAN PATENT APPLICATION

(11) **EP 4 115 929 A1**
(43) Date of publication of application: **11.01.2023**
(21) Application number: 21305962.9
(22) Date of filing: 09.07.2021
(51) Int. Cl.: A61M 16/00, A61M 16/20, A61M 16/08, A61M 16/04

(54) **HOSPITAL DEVICE FOR STIMULATING TRACHEOBRONCHIAL AIR**

(71) Applicant: Physio-Assist, 13593 Aix-en-Provence (FR)
(72) Inventor: MITHALAL, Adrien, 13593 Aix-en-Provence Cedex 3 (FR)
(74) Representative: Icosa

(57) **Abstract**

The present invention relates to a tracheobronchial-air stimulation device (1) intended to be connected to a subject comprising:
(i) a physiological interface (16) able to interface the device (1) with the subject's airways;
(ii) a vacuum interface (12) able to interface with a wall inlet (21) of a medical vacuum system (2), said vacuum interface (12) comprising a safety valve (121);
(iii) a vent (13);
(iv) an electro-valve (14) connected to said physiological interface (16), said vacuum interface (12) and said vent (13); and
(v) a control module (15) configured to control said electro-valve (14) and set a negative pressure into the physiological interface (16).

The present invention also relates to a method for stimulating tracheobronchial-air.

## Description

### FIELD OF INVENTION

The present invention pertains to the field of the treatment of obstructive ventilatory disorders. In particular, the invention relates to a device for stimulating mucus to improve its expectoration.

### BACKGROUND OF INVENTION

In the healthy individual, the lungs are covered by a "film" called mucus, whose thickness is a few millimeters and is very fluid. The function of this mucus is to protect the lung cells by isolating them from direct contact with air inspired by the lungs. The renewal of this mucus is ensured by cilia, mobile excrescences lying on the surface of the bronchi. These cilia, called vibratory, beat towards the proximal part of the airways, eliminating the inhaled particles trapped in the gel phase of the mucus which particles slide on the layer of cilia, like a conveyor belt. The clearance (the capacity of a mucociliary tissue, organ or organism to eliminate a substance from a given fluid) is carried out at a speed of 5 mm/min in the trachea, ensuring the renewal of the mucus layer every 20 minutes or so. This mucociliary clearance thus eliminates mucus to the pharynx where it will be swallowed or expectorated.

Various pathologies, such as cystic fibrosis, chronic obstructive pulmonary disease (COPD), pulmonary emphysema or chronic bronchitis, bronchiolitis, primary ciliary dyskinesia (e.g. KARTAGENER syndrome), bronchial stenosis, or sinusitis, result in an obstructive ventilatory disorder or pulmonary obstructive syndrome. These pathologies are characterized by an accumulation of mucus and result in a limitation of flow rates in the respiratory tract and by an increase in air resistance.

The resulting accumulation of bronchial mucus, because of its stagnation, is responsible for infections that can lead to serious pulmonary complications. Thus, it is necessary to perform a regular uncluttering of the lungs of patients suffering from those pathologies.

For a long time, attempts have been made to use mucolytics or mucoregulators. Unfortunately, they have shown low medical results and the treatments used nowadays are often limited to the administration of bronchodilators and respiratory physiotherapy sessions. This treatment, whose purpose is to avoid the superinfection, can be traumatic for the patient and remains of limited effectiveness when the mucus is too viscous or elastic. In order to assist patients during this treatment, various devices to facilitate the expulsion of mucus have been developed.

Such devices stimulate the tracheobronchial air of a patient by using powerful pulsations of air, vibrations of air, or positive/negative pressures. All these devices generate pressure variations during inspiration. The aim of those devices is to cause the patient to cough in order to dislodge the mucus, resulting in its expectoration. These devices have the disadvantage of causing expectoration, that is to say a cough, during the expiration of the patient. This implies a traumatic experience for the patient and a risk of collapse of the bronchial walls if the negative pressure is not sufficiently small in amplitude and of short duration.

There is thus a need for a device able to stimulate tracheobronchial air that does not involve expectoration.

There is also a need for a portable and autonomous device suitable for hospital use, *i.e.* easily installable and usable in a hospital room.

The Applicant has found that this need could be met with a device allowing passive expiration while improving the fluidification of the mucus. Indeed, the non-involvement of the respiratory muscles during expiration, due to passive expiration, avoids collapse.

### SUMMARY

The present invention relates to a tracheobronchial-air stimulation device intended to be connected to a subject comprising:
(i) a physiological interface able to interface the device with the subject's airways;
(ii) a vacuum interface able to interface with a wall inlet of a medical vacuum system, said vacuum interface comprising a safety valve;
(iii) a vent;
(iv) an electro-valve connected to said physiological interface, said vacuum interface and said vent; and
(v) a control module configured to control said electro-valve and set a negative pressure into the physiological interface.

In one embodiment, the tracheobronchial-air stimulation device does not comprise a negative pressure generator. In one embodiment, the control module is configured to control the electro-valve for the application of an expiration cycle comprising a plurality of successive expirations, each expiration having a frequency and a duty cycle, said expiration cycle comprises a first part of an expiration cycle having a first frequency and a first duty cycle, and a second part of an expiration cycle having a second frequency and a second duty cycle, wherein the first part of the expiration cycle comprises a plurality of expirations having a frequency of the order of 10 to 15 Hz and a duty cycle of 0.2 to 0.7 and the second part of an expiration cycle comprises a plurality of expirations having a frequency of the order of 4 to 7 Hz and a duty cycle of the order of 0.5 to 0.8. In one embodiment, the first part of an expiration cycle comprises a frequency of 12 Hz and a duty cycle of the order of 0.3 and the second part of an expiration cycle comprises a frequency of 6 Hz and a duty cycle of the order of 0.6. In one embodiment, the control module is configured to adapt the expiration cycle previously chosen by the operator or the subject according to the results of evaluated average stimulation duration. In one embodiment, the control module is configured to receive instructions from the subject or operator to adjust the amplitude of the departing negative pressures according to their tolerance. In one embodiment, the electro-valve is connected to the physiological interface via a connecting tube and/or the electro-valve is connected to the vacuum interface via a connecting tube. 8 In one embodiment, the safety valve is configured to close and stop immediately the expiration cycle if the vacuum generated at the wall inlet exceeds a critical threshold of -300 mbar.

The present invention also relates to a method for stimulating tracheobronchial-air comprising the steps of:
(i) providing a tracheobronchial-air stimulation device according to the invention;
(ii) connecting the vacuum interface with a wall inlet of a medical vacuum system;
(iii) connecting said tracheobronchial-air stimulation device to a subject via the physiological interface;
(iv) applying an expiration cycle comprising a plurality of successive expirations to the subject, each expiration having a frequency and a duty cycle,
wherein the expiration cycle comprises a first part of an expiration cycle having a first frequency and a first duty cycle, and a second part of an expiration cycle having a second frequency and a second duty cycle.

In one embodiment, the tracheobronchial-air stimulation device does not comprise a negative pressure generator. In one embodiment, the first part of the expiration cycle comprises a plurality of expirations having a frequency of the order of 10 to 15 Hz and a duty cycle of 0.2 to 0.7 and the second part of an expiration cycle comprises a plurality of expirations having a frequency of the order of 4 to 7 Hz and a duty cycle of the order of 0.5 to 0.8. In one embodiment, the first part of an expiration cycle comprises a frequency of 12 Hz and a duty cycle of the order of 0.3 and the second part of an expiration cycle comprises a frequency of 6 Hz and a duty cycle of the order of 0.6. In one embodiment, the control module is configured to adapt the expiration cycle previously chosen by the operator or the subject according to the results of evaluated average stimulation duration. In one embodiment, the control module is configured to receive instructions from the subject or operator to adjust the amplitude of the departing negative pressures according to their tolerance. In one embodiment, the electro-valve is connected to the physiological interface via a connecting tube and/or the electro-valve is connected to the vacuum interface via a connecting tube.

### DEFINITIONS

In the present invention, the following terms have the following meanings:
- **"Subject"** refers to a subject receiving a tracheobronchial-air stimulation using the device of the invention. The terms "subject" and "patient" can be used interchangeably herein.
- **"Expiration cycle"** refers to a plurality of successive expirations applied to a subject during a treatment session.
- **"Frequency"** refers to the number of depressions per expiration divided by the duration of the same expiration.
- **"Duty cycle"** refers to the time interval within an expiration during which the negative pressure is maintained. The duty cycle defines the relationship between the time during which negative pressure is applied to the subject and the total duration of an expiration. In other words, this duty cycle corresponds to the time during which the subject's lungs are subjected to negative pressure during an expiration.

### DETAILED DESCRIPTION

The following detailed description will be better understood when read in conjunction with the drawings. For the purpose of illustrating, the device is shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

This invention relates to a tracheobronchial-air stimulation device intended to be connected to a subject.

Said tracheobronchial-air stimulation device comprises:
(i) a physiological interface able to interface the device with the subject's airways;
(ii) a vacuum interface able to interface with a wall inlet of a medical vacuum system, said vacuum interface comprising a safety valve;
(iii) a vent;
(iv) an electro-valve connected to said physiological interface, said vacuum interface and said vent; and
(v) a control module configured to control said electro-valve and set a negative pressure into the physiological interface.

The subject typically suffers from an obstructive ventilatory disorder and the device of the invention is able to modify the rheology of his tracheobronchial mucus.

Herein, the expression "obstructive ventilatory disorders" includes pathologies such as for example cystic fibrosis, chronic obstructive pulmonary disease (COPD), pulmonary emphysema or chronic bronchitis, bronchiolitis, primary ciliary dyskinesia (e.g. KARTAGENER syndrome), bronchial stenosis, but also extends to disorders associated with nasal obstruction, such as sinusitis. Sinusitis corresponds to sinus obstruction following inflammation of the mucous membranes of the nose resulting in altered drainage of the nasal mucus.

The device targets the bronchial mucus and therefore aims at the stimulation of intra-pulmonary air in order to offer the possibility of a passive expiration while improving the fluidification of the mucus.

In particular, the device aims at aspirating air from the subject's lungs during a cycle comprising a plurality of expirations, said subject being passive. Said expiration cycle comprises a plurality of successive expirations, each expiration having eventually distinct parameters such as frequency and duty cycle. It has to be noted that during one expiration, the frequency and duty cycle are kept constant. Typically, an expiration cycle can be divided into 2 parts: a first part comprises n1 successive expirations with a frequency f1 and a duty cycle D1, then a second part comprises n2 successive expirations with a frequency f2 and a duty cycle D2, n1 and n2 being positive integers. The purpose of the first part of the expiration cycle is to fluidify and dislodge the mucus accumulated in a subject's lungs, while the purpose of the second part of the same expiration cycle is to evacuate said mucus from the subject's lungs. The number of expirations per expiration cycle is predetermined by the operator or the subject.

The passive expiration performed with the device of the present invention can provide an expiratory aid, which will increase expiratory time. The expiration is ensured by the device, thus passive for the subject, which avoids the physiological bronchial collapse which is a major obstacle to the uncluttering of the airways. This prolonged expiration allows reaching the peripheral airways, which are usually the main "target" of the uncluttering maneuvers. The uncluttering of the subject's airways is thus more effective than when using active expiration.

The medical vacuum system may be a central medical vacuum supply, *i.e.* vacuum is available from wall inlets (pipeline) located in a healthcare facility, such as for example a hospital; as such it may also be called "intramural vacuum system". The vacuum is created by running a mechanical pump like a compressor, but instead of creating a high pressure in the pump and sending that compressed gas to a receiver tank, this pump sucks the gases out of the receiver tank and forces the compressed air outside leaving a vacuum in the receiving tank that is connected to the house piping. The major source of vacuum is typically a pump, typically located in the basement of the building.

The medical vacuum system generates and delivers high vacuum at the vacuum inlet. Said high vacuum is applied to the device when said device is connected to the vacuum inlet via the vacuum interface. The control module of the device then controls the electro-valve to set a desired negative pressure to be delivered to the subject via the physiological interface. In other words, the medical vacuum system works as a vacuum provider for the device allowing said device to deliver negative pressure impulses to the lungs of a subject.

According to one embodiment, the medical vacuum system generates a vacuum ranging from 0 mbar to -900 mbar, preferably from -200 mbar to -400 mbar, more preferably from -150 mbar to -400 mbar.

The safety valve is configured to protect the fragile lungs of the subject from the vacuum generated from the medical vacuum system. Indeed, said vacuum is very strong and could damage the lungs of the subject if no valve is included in the device.

According to one embodiment, if the vacuum generated by the medical vacuum system is too strong and results in a negative pressure too strong for the lungs to support it, the risk of lungs crushing being high, the safety valve is then configured to close and thus stop immediately the expiration.

In a specific configuration of this embodiment, the safety valve is configured to close and stop immediately the expiration cycle if the vacuum generated at the wall inlet exceeds a critical threshold of -300 mbar. For example, the vacuum can exceed this critical threshold because of a malfunction of the manometer, or a bad user setting.

In an alternate configuration of this embodiment, the safety valve is configured to modify the vacuum range delivered to the device by the medical vacuum system. For example, the medical vacuum system generates a vacuum between -150 mbar and -450 mbar, and the safety valve modifies this range to be between -200 mbar and -150 mbar by applying air leaks.

In an alternate configuration of this embodiment, the safety valve is configured to close and stop immediately the expiration cycle if the negative pressure generated by the device exceeds a critical threshold of-70 mbar. Indeed, if the negative pressure applied to the lungs exceeds a critical threshold of -70 mbar, it becomes uncomfortable for the subject. In the same way, if the oscillatory flow exceeds a critical threshold of 180 L/min, the safety valve is then configured to close and thus stop immediately the expiration.

Preferably, the safety valve is a solenoid valve.

Preferably, the tracheobronchial-air stimulation device does not comprise a negative pressure generator as it uses medical vacuum system as an external negative pressure source.

According to one embodiment, the device generates an oscillatory flow rate during the patent's exhalation ranging from 20 L/min to 200 L/min, preferably from 30 L/min to 180 L/min.

According to one embodiment, negative pressure ranges from -10 mbar to -60 mbar.

According to one embodiment, the physiological interface comprises a mouthpiece or a breathing mask. Thus, the physiological interface will connect with the mouth, or the mouth and the nose of the subject wearing it.

In a preferred embodiment, said physiological interface is equipped with an RFID tag for tracking the subject.

According to one embodiment, the device comprises a connecting tube connecting the electro-valve to the physiological interface, said connecting tube being preferably flexible.

According to one embodiment, the device comprises a connecting tube connecting the electro-valve to the vacuum interface, said connecting tube being preferably flexible.

According to one embodiment, the control module is configured to adapt the expiration cycle previously chosen by the operator or the subject according to the results of evaluated average stimulation duration.

According to one embodiment, the control module is configured to receive instructions from the subject or operator to adjust the amplitude of the departing negative pressures according to their tolerance.

According to one embodiment, the expiration cycle comprises a plurality of expirations, each expiration having distinct parameters such as frequency and duty cycle.

According to one embodiment, the control module is configured to control the application of a succession of alternation of negative pressure and venting defining at least one duty cycle.

According to one embodiment, the control module is configured to control the application of a succession of negative pressure defining at least one frequency.

In one embodiment, said control module comprises a pressure sensor which is connected to the vacuum interface via a tube. In addition, said pressure sensor advantageously takes the form of a relative pressure sensor measuring the value of the vacuum with respect to the ambient atmospheric pressure. On this type of sensor, pressure fluctuations due to weather or altitude changes have a direct impact on the measured value. If the pressure exerted on the relative pressure sensor is lower than the ambient pressure, it is called negative relative pressure and the value is preceded by a sign "-".

Relative pressure sensors have a well-known structure and typically only have one pressure connection. The ambient pressure is exerted through a slot or a vent tube located at the rear of the sensor membrane and this relative measurement is compensated for. The applicant has been able to demonstrate that the negative pressure has to be maintained within a specific time interval, defined herein as duty cycle, within an expiration in order to optimize the efficiency of the treatment and avoid the variability between subjects. The control of this time makes it possible to avoid a too long expiratory time that could lead to a collapse of the bronchi. In addition, this modulation of the duty cycle of each negative pressure makes it possible, beyond the negative pressure generated which has a shearing action on the bronchial mucus and which therefore causes its viscosity to drop, to apply a flow that is longer or shorter, which improves the secretions transport. More specifically, it is important that the negative pressure applied to the tracheobronchial mucus be between -40 and -100 millibars, preferably between -45 and -80 millibars, and preferably between -30 and -70 millibars.

Preferably, the electro-valve is a solenoid valve which has a closing rate inferior to 5 ms. Said closing rate refers to the time between the between the issuance of the closing command and the effective closing of the valve.

According to one embodiment, the electro-valve is configured to tune the negative pressure amplitude by mixing the strong negative pressure provided by the medical vacuum system and the atmospheric pressure using the opening and closing of the vent.

According to one embodiment, the device further comprises a microcontroller. In this embodiment, the control module has the advantage of being able to vary the fluidification effect of the device via said microcontroller which controls the switching frequency of the electro-valve and the opening and closing times of the electro-valve. In particular, depending on the negative pressure measured by the pressure sensor and the operator-determined parameters, the microcontroller is able to control the electro-valve to mix the strong negative pressure provided by the medical vacuum system and the atmospheric pressure by opening and/or closing the vent in order to obtain a desired amplitude of negative pressure applied to the tracheobronchial mucus of the subject. The operator-determined parameters are within a range defined by a set of desirable parameters stored in the memory associated with the microcontroller. Preferably, the microcontroller is able to tune the negative pressure applied to the tracheobronchial mucus of the subject between -10 and -100 millibars, preferably between -15 and -70 millibars, more preferably between -20 and -60 millibars. In this way, the treatment applied to the subject by the device will achieve optimal efficiency and limit the impact of interpatient variability.

According to one embodiment, the first part of an expiration cycle comprises a plurality of expirations having a frequency of the order of 10 to 15 Hz and a duty cycle of 0.2 to 0.7 for mucus fluidification and the second part of an expiration cycle comprises a plurality of expirations having a frequency of the order of 4 to 7 Hz and a duty cycle of the order of 0.5 to 0.8 for mucus drainage.

In a preferred configuration of this embodiment, the first part of an expiration cycle comprises a plurality of expirations having a frequency of 12 Hz and a duty cycle of the order of 0.3 and the second part of an expiration cycle comprises a plurality of expirations having a frequency of 6 Hz and a duty cycle of the order of 0.6.

According to one embodiment, the first part of an expiration cycle comprises a plurality of expirations ranging from 2 to 10 expirations, preferably from 4 to 8 expirations, more preferably 8 expirations and the second part of an expiration cycle comprises a plurality of expirations ranging from 1 to 5 expirations, preferably from 1 to 4 expirations, more preferably 2 expirations.

Preferably, the defined number of expirations is initially selected by the subject or operator and is adapted by the device according to the results of the evaluated average stimulation duration.

According to one embodiment, the microcontroller is configured to adapt the negative pressure to obtain a negative pressure applied to the tracheobronchial mucus of the subject of between -40 and -100 millibars, preferably between -45 and -80 millibars, preferably between -50 and -100 millibars to the respiratory tract of the subject. In particular, the microcontroller is configured to control the switching frequency, the opening and closing times of the electro-valve and/or the vent.

According to one embodiment, the microcontroller is configured to also control the negative pressure provided by the device as a function of the measured duration of stimulation.

In one embodiment, the vacuum interface comprises a vacuum setting system located on the hospital room wall, said vacuum setting system is a vacuum regulator being configured to tune and/or to monitor the vacuum level. Said vacuum setting system will need to be calibrated before use. In this embodiment, the vacuum setting system may be a hospital manometer or a correspondence table comprising standard settings. Preferably, the vacuum setting system is a pressure gauge.

In another embodiment, the vacuum interface does not comprise a vacuum setting system, but is connected to the vacuum inlet via an external vacuum setting system. In this embodiment, said vacuum setting system is not included in the device of the invention.

In a specific configuration of both preceding embodiments, the vacuum inlet is connected to the safety valve of the vacuum interface via a vacuum setting system, providing this succession of elements: safety valve ― vacuum setting system - medical vacuum system.

According to one embodiment, the device further comprises a stopwatch for determining the duration of expirations during which the successive negative pressures are applied to the subject. Said stopwatch has the advantage of assisting the expiration of the subject. Thus, for an expiration to be effective, it is important for the stimulation to take place for a minimum period of at least five seconds and therefore for the expiration of subject to be maintained for at least this period. Indeed, a short time of stimulation of the subject during expiration materializes a low respiratory capacity and a descent into negative pressure too fast. This results in too strong suction of device that empties too quickly the subject's lung volume.

According to one embodiment, the device further comprises a calculator able to determine the average amplitude of negative pressure applied and the average duration of stimulation during all expirations to the subject on the basis of the negative pressure amplitude and duration measured at each expiration.

Said calculator may store in its memory each command of the electro-valve, each value of the signal from the pressure sensor and representative of the amplitude of the vacuum measured instantaneously and the number of control cycles of the electro-valve for the entire duration (determined by a scalable parameter stored by the apparatus) of the expiration cycle to allow the calculator by a calculation using the amplitude values and the expirations number to determine the average negative pressure amplitude measured at each expiration, then memorize and then determine the average negative pressure amplitude applied during all the expirations performed by the subject during the expiration cycle. The calculator associated with the pressure sensor also measures during the time intervals when the electro-valve is closed so as not to apply a negative pressure impulse, the evolution of the pressure in the lung of the subject during the expiration and the time required to reach the pressure amplitude corresponding to an expiration and stored as a threshold in the device. Said time corresponding to the duration of an expiration is stored and used by the device. This value representative of the duration of the expiration will be compared with a first threshold value, stored by the calculator which represents a value of minimum duration for the calculator to adjust the amplitude of negative pressure generated by the device when the representative value of the duration of an expiration drops below the minimum value set. A second threshold value which represents a maximum duration of the expiration and stored by the calculator is used by the latter in comparison with the measured duration to generate a control signal of the increase of the negative pressure if the duration of the expiration measured exceeds the second saved value.

In some embodiments, the device includes a dead man remote control, which must be actively and continuously actuated or regularly reset by the subject, during the treatment, for the device to operate. Otherwise, the device stops.

In a preferred embodiment, an internal power supply supplies the various components of the device such as the pressure sensor, the stopwatch and/or the microcontroller. In this embodiment, the internal power supply may be a battery.

In one embodiment, an external power supply supplies the various components of the device such as the pressure sensor, the stopwatch and/or the microcontroller.

In another aspect, the present invention relates to a method for stimulating tracheobronchial-air comprising the steps of:
(i) providing a tracheobronchial-air stimulation device of the invention;
(ii) connecting the vacuum interface with a wall inlet of a medical vacuum system;
(iii) connecting said tracheobronchial-air stimulation device to a subject via the physiological interface;
(iv) applying an expiration cycle comprising a plurality of successive expirations to the subject, each expiration having a frequency and a duty cycle,
wherein the expiration cycle comprises a first part of an expiration cycle having a first frequency and a first duty cycle, and a second part of an expiration cycle having a second frequency and a second duty cycle.

The tracheobronchial-air stimulation device is as described hereabove.

In one embodiment, after connecting the vacuum interface with a wall inlet of a medical vacuum system (step ii), the vacuum is regulated via a vacuum setting system.

In one embodiment, the first part of the expiration cycle comprises a plurality of expirations having a frequency of the order of 10 to 15 Hz and a duty cycle of 0.2 to 0.7 and the second part of an expiration cycle comprises a plurality of expirations having a frequency of the order of 4 to 7 Hz and a duty cycle of the order of 0.5 to 0.8.

In a specific configuration of this embodiment, the first part of an expiration cycle comprises a frequency of 12 Hz and a duty cycle of the order of 0.3 and the second part of an expiration cycle comprises a frequency of 6 Hz and a duty cycle of the order of 0.6.

While various embodiments have been described and illustrated, the detailed description is not to be construed as being limited hereto. Various modifications can be made to the embodiments by those skilled in the art without departing from the true spirit and scope of the disclosure as defined by the claims.

### BRIEF DESCRIPTION OF THE DRAWINGS

**Figure 1** represents the tracheobronchial-air stimulation device according to one embodiment.
**Figure 2** represents the tracheobronchial-air stimulation device connected to the subject.
**Figure 3A** represents an example of the negative pressure signals generated by the device when the vacuum level of the medical vacuum system is set to -150 mbar.
**Figure 3B** shows the negative pressure signals of figure 3A in the time range from 4 to 5 seconds.
**Figure 3C** represents an example of the flow rate signals generated by the device when the vacuum level of the medical vacuum system is set to -150 mbar.
**Figure 3D** shows the flow rate signals of figure 3C in the time range from 4 to 5 seconds.
**Figure 4A** represents an example of the negative pressure signals generated by the device when the vacuum level of the medical vacuum system is set to -200 mbar.
**Figure 4B** represents an example of the flow rate signals generated by the device when the vacuum level of the medical vacuum system is set to -200 mbar.
**Figure 5A** represents an example of the negative pressure signals generated by the device when the vacuum level of the medical vacuum system is set to -250 mbar.
**Figure 5B** represents an example of the flow rate signals generated by the device when the vacuum level of the medical vacuum system is set to -250 mbar.
**Figure 6A** represents an example of the negative pressure signals generated by the device when the vacuum level of the medical vacuum system is set to -300 mbar.
**Figure 6B** represents an example of the flow rate signals generated by the device when the vacuum level of the medical vacuum system is set to -300 mbar.

### ILLUSTRATIVE EMBODIMENTS OF THE INVENTION

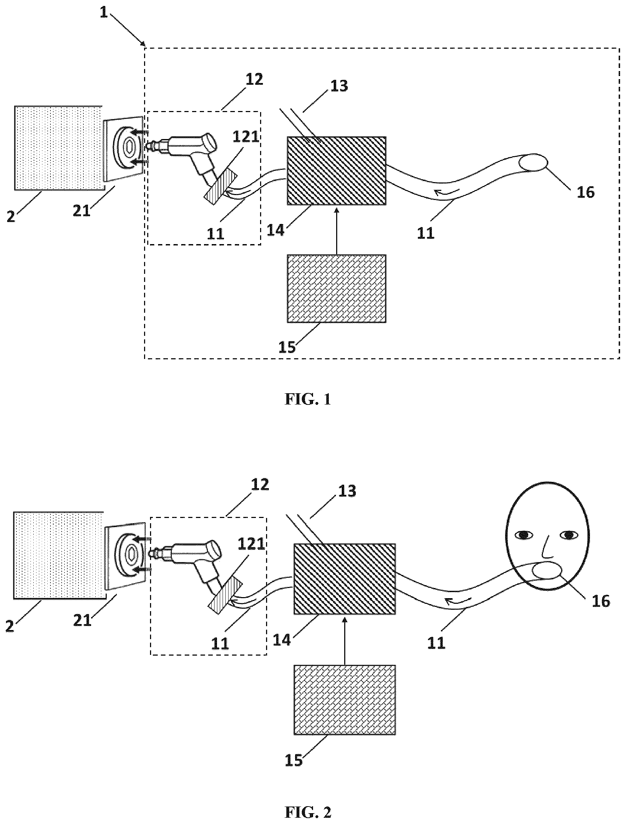

As shown in Figures 1 and 2, the tracheobronchial-air stimulation device **1** comprises:
(i) a physiological interface **16** interfacing the device with a subject's airways;
(ii) a vacuum interface **12** interfacing with a wall inlet **21** of a medical vacuum system **2,** said vacuum interface **12** comprising a safety valve **121;**
(iii) a vent **13;**
(iv) an electro-valve **14** connected to said physiological interface **16,** said vacuum interface **12** and said vent **13;** and
(v) a control module **15** configured to control said electro-valve **14** and set a negative pressure into the physiological interface **16.**

In this embodiment, vacuum is provided by the medical vacuum system **2.** The device **1** is connected to said medical vacuum system **2** by the wall inlet **21.** The vacuum interface **12** interfaces with said wall inlet **21** using a flexible pipe **11** to provide vacuum for the device **1.** The safety valve **121** of the vacuum interface **12** is configured to ensure the safety of the subject's lungs in regard to the vacuum provided. If said vacuum is too strong and results in a negative pressure too strong for the lungs to support it, the risk of lungs crushing being high, the safety valve **121** is then configured to close and thus stop the expiration. The control module **15** controls said electro-valve **14,** which opens and closes at a frequency chosen by the operator or the subject, and sets a negative pressure into the physiological interface **16** to ensure passive expiration cycles leading to the expectoration of mucus from the subject.

This embodiment is particularly advantageous as it allows passive expiration while improving the fluidification of the mucus. Indeed, the expiration is ensured by the device 1, thus passive for the subject, which avoids the physiological bronchial collapse which is a major obstacle to the uncluttering of the airways.

### EXAMPLES

The present invention is further illustrated by the following examples.

### Example 1: Validation test

A validation test was conducted on the device of the invention to ensure that said device is suitable for use on a subject. The validation test was not performed on a subject.

The validation criteria are the following:
- the negative pressure should stay within the interval of -300 mbar to -10 mbar;
- the flow rate should stay within the interval of 30 L/min to 180 L/min.

A device according to the invention is set up.

The vacuum interface is connected to the wall inlet of the medical vacuum system. The vacuum level from the medical vacuum system is tuned prior use.

The flow rate and negative pressure delivered by the device are then measured.

Flow rate measurements were performed in an open loop (physiological interface left open), and negative pressure measurements were performed in a close loop (physiological interface obstructed). Measurements were performed ex vivo, *i.e.* not on a subject.

Table 1 below lists configurations in which experiments have been performed.

**Table 1**

| Vacuum level at wall inlet (mbar) | First part of the expiration cycle | | | Second part of the expiration cycle | | | Results (negative pressure and flow rate) | |
|---|---|---|---|---|---|---|---|---|
| / | Number of expirations (n1) | Frequency (Hz) | Duty cycle | Number of expirations (n2) | Frequency (Hz) | Duty cycle | Maximum negative pressure (mbar) | Maximum oscillatory flow rate (L/min) |
| -150 | 8 | 12 | 40% | 2 | 6 | 40% | -81 | 63.9 |
| -200 | 8 | 12 | 40% | 2 | 6 | 40% | -136 | 76.2 |
| -250 | 8 | 12 | 40% | 2 | 6 | 40% | -155 | 85.5 |
| -300 | 8 | 12 | 40% | 2 | 6 | 40% | -175 | 96.1 |

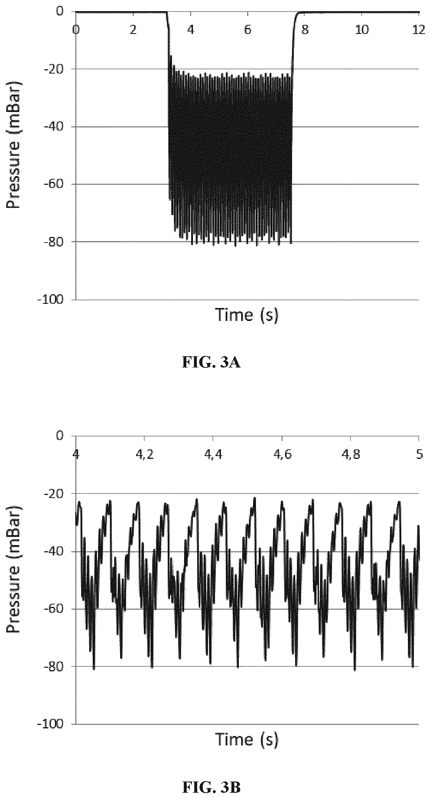

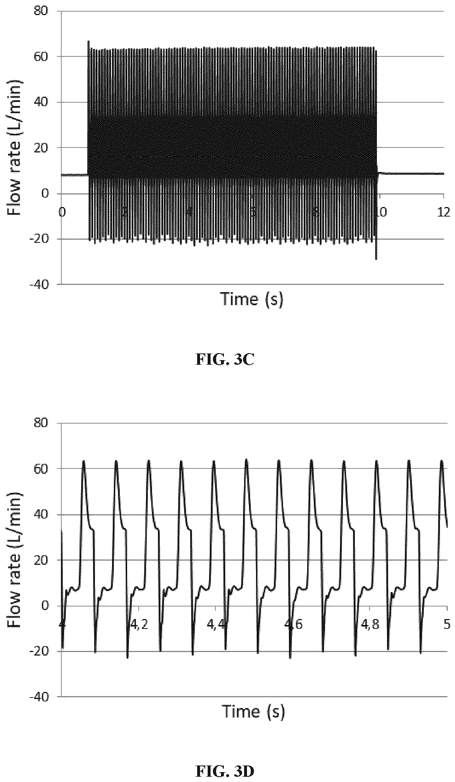

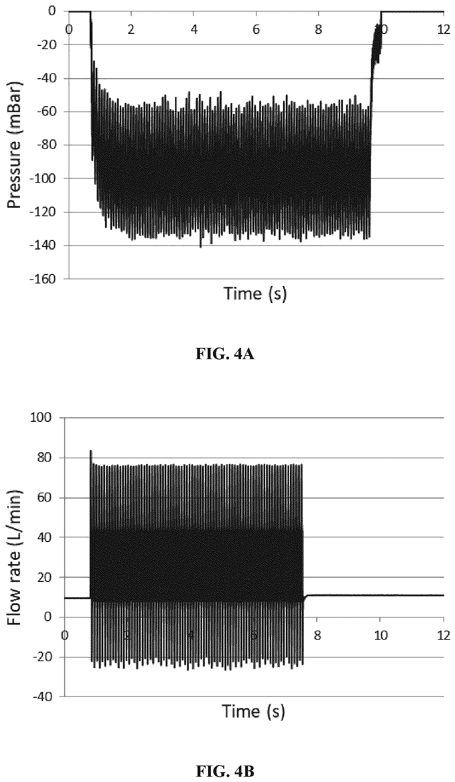

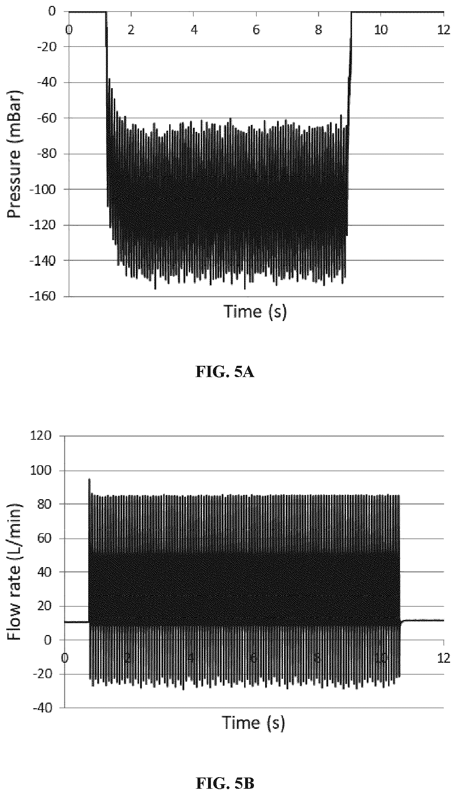

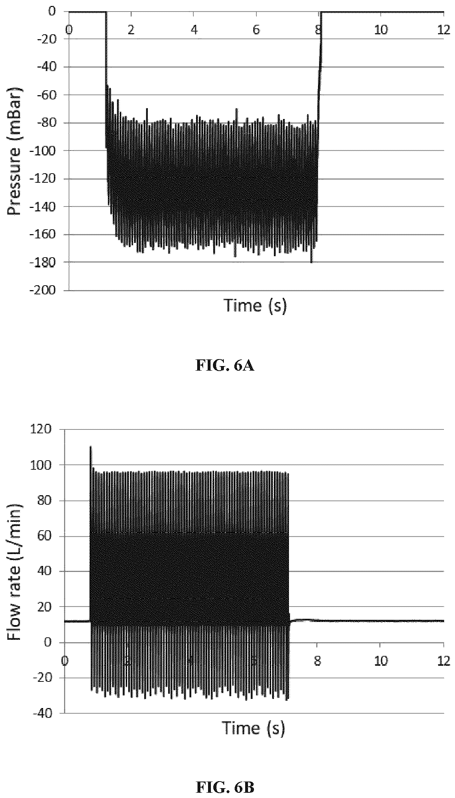

As observed on figures 3-6, the negative pressure stayed within the interval of:
- Lower limit: -300 mbar
- Upper limit: -30 mbar

The flow rate stayed within the interval of:
- Lower limit: 30 L/min
- Upper limit: 180 L/min

No deviations were observed during testing.

Figures 3B and 3D show the impulsions of negative pressure delivered by the device between 4 and 5 seconds. In this case, there were 12 impulsions in 1 second of experiment.

Thus, validation criteria were fully met in all experiments, indicating that the device of the invention is safe to use on a subject.

### Example 2: Mucus evacuation

A device according to the invention is set up.

The vacuum interface is connected to the wall inlet of the medical vacuum system. The vacuum level from the medical vacuum system is tuned prior use.

The physiological interface is adapted on the subject.

The parameters of the expiration cycle (number of expirations, negative pressure, frequency, duty cycle) are set up by the operator or the subject.

Table 2 below lists configurations in which experiments have been performed.

**Table 2**

| Vacuum level at the wall inlet (mbar) | Negative pressure delivered by the device (mbar) | First part of the expiration cycle | | | Second part of the expiration cycle | | | Treatment efficiency |
|---|---|---|---|---|---|---|---|---|
| / | / | Number of expirations (n1) | Frequency (Hz) | Duty cycle | Number of expirations (n2) | Frequency (Hz) | Duty cycle | |
| -150 | -50 | 8 | 12 | 40% | 2 | 6 | 40% | Mucus evacuated |
| -150 | -50 | 3 | 6 | 20% | 5 | 4 | 30% | Mucus non evacuated |

With the first part of the expiration cycle comprising a plurality of expirations having a frequency of the order of 10 to 15 Hz and a duty cycle of 0.2 to 0.7 and the second part of the expiration cycle comprising a plurality of expirations having a frequency of the order of 4 to 7 Hz and a duty cycle of the order of 0.5 to 0.8, the mucus is efficiently evacuated.

This relies on the efficiency of the first part of the expiration cycle to fluidify the mucus, allowing the second part of the expiration cycle to drain the fluidified mucus.

When parameters such as frequency and duty cycle do not fall within the precited ranges, the mucus is not evacuated.

### REFERENCES

1 ― Tracheobronchial-air stimulation device
11 ― Pipe
12 ― Vacuum interface
121 ― Safety valve
13 ― Vent
14 ― Electro-valve
15 ― Control module
16 ― Physiological interface
2 ― Medical vacuum system
21 ― Wall inlet

## Claims

1. A tracheobronchial-air stimulation device (1) intended to be connected to a subject comprising:
(i) a physiological interface (16) able to interface the device (1) with the subject's airways;
(ii) a vacuum interface (12) able to interface with a wall inlet (21) of a medical vacuum system (2), said vacuum interface (12) comprising a safety valve (121);
(iii) a vent (13);
(iv) an electro-valve (14) connected to said physiological interface (16), said vacuum interface (12) and said vent (13); and
(v) a control module (15) configured to control said electro-valve (14) and set a negative pressure into the physiological interface (16).

2. The tracheobronchial-air stimulation device (1) according to claim 1, which does not comprise a negative pressure generator.

3. The device (1) according to any one of claim 1 or 2, wherein the control module is configured to control the electro-valve for the application of an expiration cycle comprising a plurality of successive expirations, each expiration having a frequency and a duty cycle, said expiration cycle comprises a first part of an expiration cycle having a first frequency and a first duty cycle, and a second part of an expiration cycle having a second frequency and a second duty cycle, wherein the first part of the expiration cycle comprises a plurality of expirations having a frequency of the order of 10 to 15 Hz and a duty cycle of 0.2 to 0.7 and the second part of an expiration cycle comprises a plurality of expirations having a frequency of the order of 4 to 7 Hz and a duty cycle of the order of 0.5 to 0.8.

4. The device (1) according to claim 3, wherein the first part of an expiration cycle comprises a frequency of 12 Hz and a duty cycle of the order of 0.3 and the second part of an expiration cycle comprises a frequency of 6 Hz and a duty cycle of the order of 0.6.

5. The device (1) according to any one of claims **1** to **4,** wherein the control module (15) is configured to adapt the expiration cycle previously chosen by the operator or the subject according to the results of evaluated average stimulation duration.

6. The device (1) according to any one of claims **1** to **5,** wherein the control module (15) is configured to receive instructions from the subject or operator to adjust the amplitude of the departing negative pressures according to their tolerance.

7. The device (1) according to any one of claims **1** to **6,** wherein the electro-valve (14) is connected to the physiological interface (16) via a connecting tube (11) and/or the electro-valve (14) is connected to the vacuum interface (12) via a connecting tube (11).

8. The device (1) according to any one of claims **1** to **7,** wherein the safety valve is configured to close and stop immediately the expiration cycle if the vacuum generated at the wall inlet exceeds a critical threshold of-300 mbar.

9. A method for stimulating tracheobronchial-air comprising the steps of:
(i) providing a tracheobronchial-air stimulation device (1) according to any one of claims **1** to **8;**
(ii) connecting the vacuum interface (12) with a wall inlet (21) of a medical vacuum system (2);
(iii)connecting said tracheobronchial-air stimulation device (1) to a subject via the physiological interface (16);
(iv)applying an expiration cycle comprising a plurality of successive expirations to the subject, each expiration having a frequency and a duty cycle,
wherein the expiration cycle comprises a first part of an expiration cycle having a first frequency and a first duty cycle, and a second part of an expiration cycle having a second frequency and a second duty cycle.

10. The method according to claim **9,** wherein the tracheobronchial-air stimulation device (1) does not comprise a negative pressure generator.

11. The method according to any one of claim **9** or **10,** wherein the first part of the expiration cycle comprises a plurality of expirations having a frequency of the order of 10 to 15 Hz and a duty cycle of 0.2 to 0.7 and the second part of an expiration cycle comprises a plurality of expirations having a frequency of the order of 4 to 7 Hz and a duty cycle of the order of 0.5 to 0.8.

12. The method according to claim **11,** wherein the first part of an expiration cycle comprises a frequency of 12 Hz and a duty cycle of the order of 0.3 and the second part of an expiration cycle comprises a frequency of 6 Hz and a duty cycle of the order of 0.6.

13. The method according to any one of claims **9** to **12,** wherein the control module (15) is configured to adapt the expiration cycle previously chosen by the operator or the subject according to the results of evaluated average stimulation duration.

14. The method according to any one of claims **9** to **13,** wherein the control module (15) is configured to receive instructions from the subject or operator to adjust the amplitude of the departing negative pressures according to their tolerance.

15. The method according to any one of claims **9** to **14,** wherein the electro-valve (14) is connected to the physiological interface (16) via a connecting tube (11) and/or the electro-valve (14) is connected to the vacuum interface (12) via a connecting tube (11).
